# EUROPÄISCHE PATENTSCHRIFT

(11) **EP 2 620 223 B1**
(45) Veröffentlichungstag und Bekanntmachung des Hinweises auf die Patenterteilung: **04.03.2015**
(21) Anmeldenummer: 13152436.5
(22) Anmeldetag: 23.01.2013
(51) Int. Cl.: A61M 11/00, A61M 11/06, A61M 11/08, A61M 15/00, B05B 1/14, B05B 1/34

(54) **Düseneinheit und Spender mit einer solchen**
Nozzle unit and dispenser comprising such a nozzle
Unité à buses et distributeur en étant équipé

(30) Priorität: 27.01.2012 DE 102012201178
(43) Veröffentlichungstag der Anmeldung: 31.07.2013
(73) Patentinhaber: Aptar Radolfzell GmbH, 78315 Radolfzell (DE)
(72) Erfinder: Jung, Timo, 78244 Gottmadingen (DE)
(74) Vertreter: Patentanwälte Ruff, Wilhelm, Beier, Dauster & Partner

(56) Entgegenhaltungen:
- FR-A1- 2 798 068
- US-A- 2 994 483
- US-A- 5 421 519
- US-A- 5 516 045
- US-A- 5 735 465
- US-A- 5 785 250
- US-B1- 6 516 795

## Beschreibung

Die Erfindung betrifft eine Düseneinheit nach dem Oberbegriff von Anspruch 1. Die Erfindung betrifft weiterhin auch einen Spender mit einer solchen Düseneinheit.

Gattungsgemäße und erfindungsgemäße Spender dienen dem Austrag pharmazeutischer Medien. Während die meisten bekannten Spender zum Austrag pharmazeutischer Medien lediglich eine Austragdüse mit einer Austragöffnung aufweisen, besteht in Einzelfällen auch der Bedarf, durch mehrere Austragöffnungen mehrerer Austragdüsen gleichzeitig das pharmazeutische Medium auszutragen. Spender die hierfür ausgebildet sind, sind beispielsweise aus der EP 0786421 A1, der US 3,161,196 und der FR 2467604 A1 bekannt. Bei diesen gattungsgemäßen Spendern sind mehrere Austragöffnungen unmittelbar verbunden mit dem gemeinsamen Zuführkanal vorgesehen.

Allerdings hat sich gezeigt, dass die Verabreichung pharmazeutischer Medien mit derartigen Spendern in der Regel nicht zum gewünschten Erfolg führt, da das Medium bei den bekannten Vorrichtungen in weitgehend unzerstäubter Form ausgebracht wird.

Aus dem Stand der Technik ist auch eine Vielzahl von Spendern bekannt, die Verwirbelungseinrichtungen aufweisen, durch die das Medium vor dem Austrag verwirbelt wird. Diese Verwirbelungseinrichtungen verfügen über eine Wirbelkammer, in die das Medium derart eingebracht wird, dass es dort und damit vor seinem Austrag mit einem Drall versehen wird, der einen Austrag in Form eines konischen Sprühstrahls gestattet.

Um eine ähnlich gute Austragqualität auch bei gattungsgemäßen Spendern mit mehr als einer Austragöffnung zu erzielen, wurde versucht, den Austragöffnungen eine gemeinsame Wirbelkammer vorzuschalten. Es hat sich allerdings gezeigt, dass dies zu einem komplexen und schwer herzustellenden Aufbau führt, wobei zudem keine zufriedenstellende Qualität des Sprühstrahls erzielbar zu sein scheint.

Aus dem Stand der Technik sind auch Dokumente bekannt, die bereits Spender mit divergierender Austragrichtung offenbaren, so beispielsweise die US 5,516,045 A, die FR 2798068 A1 und die US 5,785,250 A. Soweit die dort vorgestellten Spender Wirbelkammern aufweisen, sind diese zu den jeweiligen Austragrichtungen der Austragöffnungen koaxial ausgerichtet.

### Aufgabe und Lösung

Aufgabe der Erfindung ist es daher, eine gattungsgemäße Düseneinheit dahingehend weiterzubilden, dass diese zur Ausbringung mindestens zwei qualitativ guter Sprühstrahlen ausgebildet ist und vorteilhaft herstellbar ist. Aufgabe der Erfindung ist es weiterhin, einen Spender mit einer solchen Düseneinheit zur Verfügung zu stellen.

Erfindungsgemäß wird die zugrunde liegende Aufgabe mit einer Düseneinheit nach Anspruch 1 erzielt.

Erfindungsgemäß ist somit vorgesehen, dass beiden Austragöffnungen jeweils eine eigene Wirbelkammer vorgeschaltet ist, der jeweils Medium aus dem gemeinsamen Zuführkanal zugeführt wird. Innerhalb dieser Wirbelkammern wird das zugeführte Medium mit dem gewünschten Drall versehen und durch die beiden Austragöffnungen ausgetragen.

Obwohl bislang und im weiteren schwerpunktartig auf eine Gestaltung Bezug genommen wird, bei der zwei Austragöffnungen und damit auch zwei Wirbelkammern vorgesehen sind, können eine erfindungsgemäße Düseneinheit sowie ein erfindungsgemäßer Spender selbstverständlich auch mit weiteren Austragdüsen mit weiteren Austragöffnungen und weiteren Wirbelkammern versehen sein. Es gilt dabei für alle Austragdüsen, dass diese jeweils eine Austragöffnung und eine eigene dieser Austragöffnung vorgeschaltete Wirbelkammer aufweist.

Eine Wirbelkammer im Sinne der vorliegenden Erfindung ist eine Kammer, die durch gegeneinander unbewegliche Teile definiert wird und die mindestens einen Einlass und einen in Richtung der Austragöffnung gerichteten Auslass aufweist. Die Formgebung dieser Wirbelkammer ist dergestalt, dass das flüssiges Medium, welches unter Überdrück einlassseitig in die Wirbelkammer gelangt, hier mit einem Drall versehen wird, bevor es in Richtung des Auslasses aus der Wirbelkammer herausströmt. Der Drall wird vorzugsweise durch eine etwa kreiszylindrische Form der Wirbelkammer und eine von der Radialrichtung abweichende Einströmrichtung des Mediums bewirkt. Auf die Achse, um die das Medium in der Wirbelkammer rotiert und die im Falle kreiszylindrischer Wirbelkammern mit der Zylinderachse übereinstimmt, wird im Weiteren unter dem Begriff der Wirbelkammerachse Bezug genommen.

Durch die erfindungsgemäß vorgesehene Gestaltung mit jeweils einer Wirbelkammer je Austragdüse kann in der bereits genannten Weise das vorteilhafte Sprühbild erzeugt werden. Da jeweils nur eine Austragöffnung durch das in einer Wirbelkammer mit einem Drall versehene Medium gespeist wird, sind keine Kompromisse hinsichtlich der Zuführung des Mediums von der Wirbelkammer zur Austragöffnung erforderlich.

Allerdings geht die Gestaltung mit einer Mehrzahl von Wirbelkammern grundsätzlich mit dem Problem erhöhter Herstellungskosten einher.

Es wird daher insbesondere unter dem Gesichtspunkt einer kostengünstigen Herstellung als vorteilhaft angesehen, wenn die Düseneinheit ein Basisbauteil aufweist, in welchem zumindest abschnittsweise der Zuführkanal vorgesehen ist, die Düseneinheit weiterhin mindestens ein Aufsatzbauteil aufweist, welches zumindest die Austragöffnung der ersten Austragdüse aufweist und die erste Wirbelkammer der ersten Austragdüse durch Wandungen des Basisbauteils und durch Wandungen des Aufsatzbauteils begrenzt wird.

Die erfindungsgemäße Düseneinheit umfasst somit vorzugsweise ein Bauteil, das Basisbauteil, welches zumindest einen endseitigen Teil des Zuführkanals bildet. Dieses Basisbauteil begrenzt gemeinsam mit einem zweiten Bauteil, dem Aufsatzbauteil, mindestens eine der Wirbelkammern. Da diese bis auf die genannten Einlässe und den genannten Auslass nach außen durch Wandungen begrenzt sein muss, müssen aus fertigungstechnischen Gründen mindestens zwei Bauteile Verwendung finden. Gemäß dieser Weiterbildung sind dies jene beiden Bauteile, welche zur Bildung des Zuführkanals und zur Bildung der Austragöffnungen ohnehin vorgesehen sein müssen.

Somit kann eine erfindungsgemäße Düseneinheit im Extremfalle und im Idealfalle aus nur zwei Bauteilen bestehen, nämlich dem Basisbauteil und dem Aufsatzbauteil.

Zwar ist es grundsätzlich möglich, mehrere Aufsatzbauteile zu verwenden, die jeweils über eine Austragöffnung verfügen und eine Wirbelkammer gemeinsam mit dem Basisbauteil definieren. Von Vorteil ist jedoch eine Gestaltung mit nur einem Aufsatzbauteil, welches mehrere, vorzugsweise alle, Austragöffnungen der Austragdüsen umfasst, und vorzugsweise ebenso viele Wirbelkammern begrenzt.

Das Aufsatzbauteil und das Basisbauteil sind vorzugsweise dafür ausgebildet, unmittelbar durch eine kraftschlüssige oder eine formschlüssige Verbindung miteinander verbunden zu werden. Hier bieten sich Pressverbindungen und Schnappverbindungen an. Auch eine stoffschlüssige Verbindung durch Verschweißen der Bauteile oder Verkleben der Bauteile ist grundsätzlich möglich. Als Material für das Basisbauteil und das oder die Aufsatzbauteile ist in der Regel Kunststoff zweckmäßigerweise zu verwenden. Die Bauweise der Düseneinheit gestattet es, die beiden Kunststoffteile, die das Aufsatzbauteil und das Basisbauteil der Düseneinheit bilden, in einfach entformbarer Weise als Kunststoffspritzgussteile zu fertigen.

Wirbelkammern für erfindungsgemäße Düseneinheiten verfügen über eine Strömungsleitgeometrie, die derart geformt ist, dass sie in der Lage ist, das Medium umzulenken mit dem genannten Drall zu versehen. Wie bereits erläutert, umfassen diese Strömungsleitgeometrien hierfür insbesondere eine kreiszylindrische Wandung oder anderweitige gekrümmte Wandungen, entlang derer das Medium bei gleichzeitiger Richtungsänderung entlangströmt. Als besonders vorteilhaft wird es angesehen, wenn diese zur Umlenkung des Medienstroms geeigneten Strömungsleitgeometrien an einer Innenseite des Aufsatzbauteils vorgesehen sind. So kann an einer zum Basisteil weisenden Innenseite des Aufsatzbauteils eine Fläche vorgesehen sein, die nach Montage bündig an dem Basisbauteil anliegt, wobei in dieser Fläche Vertiefungen vorgesehen sind, die die Strömungsleitgeometrien bilden. Eine solche Bauweise ist im Zuge der Herstellung des Aufsatzbauteils besonders einfach zu erzielen. Dennoch ist es grundsätzlich auch möglich, die entsprechenden Geometrien statt am Aufsatzbauteil am Basisbauteil und dort insbesondere an einer stirnseitigen Wandung vorzusehen. Ebenso wie am Aufsatzbauteil können die Strömungsleitgeometrien dabei dadurch erzielt werden, dass in einer am jeweiligen Bauteil vorgesehenen Fläche Vertiefungen vorgesehen sind, wodurch die Vertiefungen umgebende Bereiche gegenüber den Vertiefungen erhaben sind und Teil der Strömungsleitgeometrie bilden können.

Im Zusammenhang mit der Gestaltung des Aufsatzbauteils mit den Strömungsleitgeometrien wird es weiterhin als besonders vorteilhaft angesehen, wenn das Basisbauteil zumindest in einem an das Aufsatzbauteil angrenzenden Bereich durch einen Rohrabschnitt gebildet ist, wobei eine ringförmige Stirnfläche dieser Rohrabschnitts die erste und die zweite Wirbelkammer begrenzt. Vorzugsweise ist der genannte Rohrabschnitt mit einem kreisförmigen Querschnitt ausgebildet. Die Wirbelkammern sind am Aufsatzbauteil vorzugsweise derart angeordnet, dass sie zwischen dem Innendurchmesser und dem Außendurchmesser des genannten Rohrabschnitts angeordnet sind, so dass dessen Stirnfläche die Wirbelkammern verschließen kann. Dabei muss die Stirnfläche nicht zwingend vollständig eben ausgebildet sein. Sie kann auch anderweitige Formgebungen aufweisen, beispielsweise auch die Form eines Konusabschnitts.

Die Austragrichtung durch die erste Austragdüse und die Austragrichtung durch die zweite Austragdüse schließen einen Winkel > 10° und < 150° ein und divergieren somit. Für den insbesondere relevanten Anwendungszweck der Rachensprühspender wird ein Winkel zwischen 45° und 90° als ideal angesehen. Unter der Austragrichtung wird im Zusammenhang mit dieser Erfindung die Mittelachse des sich vorzugsweise einstellenden Sprühkegels verstanden. Die konkrete Ausrichtung der Austragrichtungen ist in Abhängigkeit des Anwendungszwecks auszuwählen.

Erfindungsgemäß wird das Medium zwischen der Wirbelkammer und der Austragöffnung nochmals umgelenkt. Dies gestattet es insbesondere, die Wirbelkammern derart auszulegen, dass ihrer jeweiligen Wirbelkammerachsen zueinander parallel verlaufen. Dies ist im Zusammenhang mit einer planen Stirnfläche des Basisbauteils ideal. Es erleichtert zudem die Entformung im Zuge der Herstellung insbesondere des Aufsatzbauteils.

Wie eingangs bereits erläutert, werden die Wirbelkammern über den Zuführkanal mit Medium gespeist. Hierfür ist mindestens ein Einlasskanal erforderlich, der den Zuführkanal mit der Wirbelkammer verbindet und der vorzugsweise nicht-radial, insbesondere tangential, in die Wirbelkammer mündet. Zur Verbesserung des Sprühstrahls ist bei einer bevorzugten Variante vorgesehen, dass zumindest im Hinblick auf eine der Wirbelkammern zumindest zwei Einlasskanäle vorgesehen sind, die in diese Wirbelkammer münden. Diese gehen dabei vorzugsweise von einem die Wirbelkammer umgebenden Ringkanal ab, der seinerseits vom Zuführkanal gespeist wird. Durch zwei Einlasskanäle kann erreicht werden, dass das Medium gleichmäßiger in die Wirbelkammer eindringt und somit zu einem verbesserten Sprühstrahl führt. Der umgebende Ringkanal leistet in besonders vorteilhafter Weise die gleichmäßige Speisung der Wirbelkammer durch die mindestens zwei Einlasskanäle. Grundsätzlich ist natürlich auch ohne eine Ringkammer die Speisung von Mehr als einem Einlasskanal in dieselbe Wirbelkammer möglich.

Die Erfindung betrifft weiterhin auch einen Spender zum Austrag von pharmazeutischen Medien in zerstäubter Form. Dieser weist erfindungsgemäß eine Düseneinheit vorbeschriebener Art auf.

Insbesondere kann ein erfindungsgemäßer Spender als Rachensprühspender ausgebildet sein und zu diesem Zweck einen rohrartigen und sich vorzugsweise radial zu einer Betätigungsrichtung des Spenders erstreckenden Abschnitt mit einer Länge von vorzugsweise mindestens 20 mm aufweisen, der in den Mund des Patienten eingeführt wird. Dieser rohrartige Abschnitt bildet vorzugsweise das Basisbauteil der beschriebenen Düseneinheit. Je nach Anwendungszweck können insbesondere auch Längen von mehr als 30mm oder sogar mehr als 40mm zweckmäßig sein.

Bei einem solchen Rachsprühspender kann durch die beiden Austragdüsen und die dirigierende Ausrichtung des durch die beiden Düsen stattfindenden Austrags erreicht werden, dass der Austrag links und rechts am Gaumenzäpfchen des Patienten vorbei erfolgt. Dies ist für den Patienten angenehmer und dient daher letzten Endes auch der korrekten Verabreichung des pharmazeutischen Mediums.

Beim pharmazeutischen Medium selbst kann es sich vorzugsweise um ein Medikament gegen Erkältungskrankheiten handeln.

### Kurzbeschreibung der Zeichnungen

Weitere Aspekte und Vorteile der Erfindung ergeben sich aus den Ansprüchen sowie der nachfolgenden Beschreibung von bevorzugten Ausführungsbeispielen der Erfindung, die anhand der Figuren erläutert werden. Dabei zeigen:
- Fig. 1a und 1b: einen erfindungsgemäßen Spender bzw. dessen Austragkopf,
- Fig. 2a bis 2d: Ausgestaltungen des Austragkopfes der Fig. 1a und 1b mit einer ersten Ausgestaltung einer erfindungsgemäßen Düseneinheit,
- Fig. 3a bis 3c: Ausgestaltungen des Austragkopfes der Fig. 1a und 1b mit einer zweiten Ausgestaltung einer erfindungsgemäßen Düseneinheit und
- Fig. 4a: Ausgestaltungen des Austragkopfes der Fig. 1a und 1b mit einer dritten Ausgestaltung einer erfindungsgemäßen Düseneinheit.

### Detaillierte Beschreibung der Ausführungsbeispiele

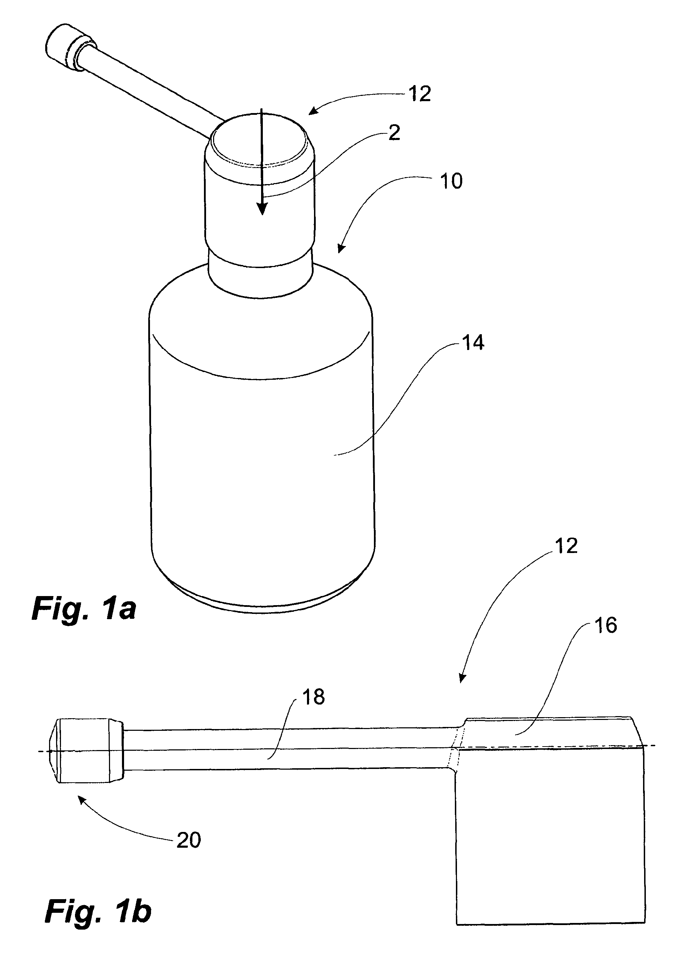

Die Fig. 1a und 1b zeigen einen erfindungsgemäßen Spender 10 mit einem Austragkopf 12. Der Spender 10 verfügt über ein Reservoir 14, welches der Lagerung eines pharmazeutischen Mediums vor dessen Austrag dient. Der Austragkopf 12 ist in seiner Gesamtheit gegenüber dem Reservoir 14 in Richtung einer Betätigungsrichtung 2 verlagerbar, wobei hierdurch eine nicht dargestellte Pumpeinrichtung betätigt wird, die Medium aus dem Reservoir 14 in den Austragkopf 12 fordert.

Der Austragkopf 12 ist in Fig. 1b in einer Seitenansicht dargestellt. Er umfasst eine als Drücker ausgebildete Betätigungshandhabe 16 sowie einen langerstreckten rüsselartigen Applikator 18, an dessen Ende eine Düseneinheit 20 zur zerstäubten Abgabe des Mediums vorgesehen ist.

Nachfolgend werden verschiedene Varianten zur Ausgestaltung insbesondere der Düseneinheit 20 vorgestellt.

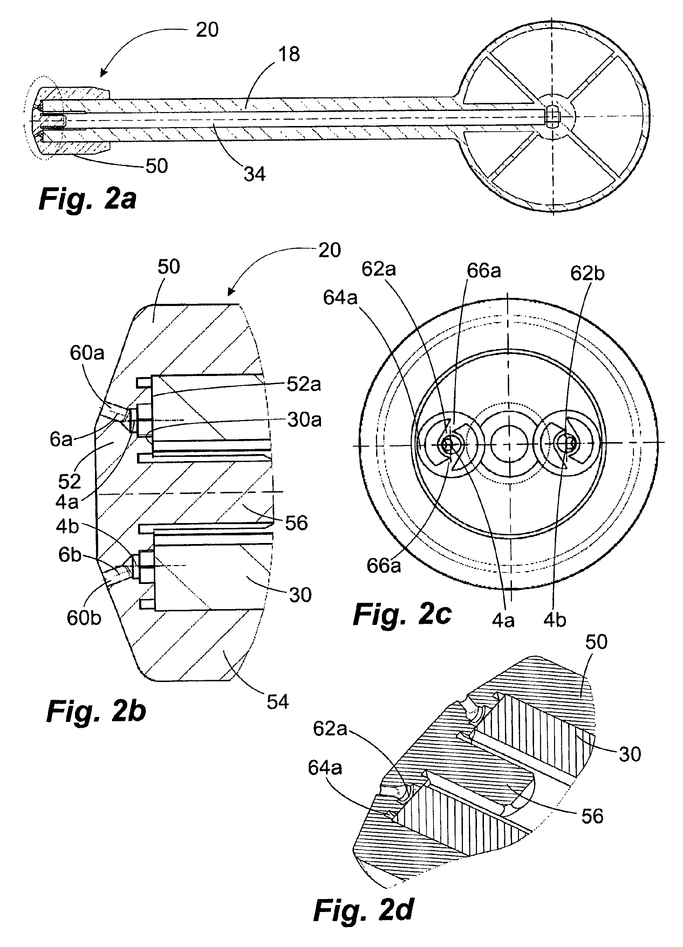

Die anhand der Fig. 2a bis 2c verdeutlichte Düseneinheit 20 wird durch ein ein Basisbauteil darstellendes Rohr 30 des Applikators 18 sowie ein endseitig auf dieses Rohr 30 aufgeschobenes Aufsatzbauteil 50 gebildet. Das Aufsatzbauteil 50 weist einen hülsenartigen Aufbau mit einem von einer Stirnfläche 52 ausgehenden Kragen 54 auf, der beispielsweise kraftschlüssig auf der Außenseite des Rohrs 30 befestigt sein kann. Weiterhin weist das Aufsatzbauteil 50 einen zentrischen und sich in das Rohr 30 erstreckenden stiftartigen Fortsatz 56 auf.

Die Stirnseite 52 ist durch zwei Austragöffnungen 60a, 60b durchdrungen. An der Innenseite 52a der Stirnfläche 52 sind die Austragöffnungen 60 umgebend verschiedene Vertiefungen vorgesehen. Diese umfassen in der insbesondere Fig. 2c entnehmbaren Form vor allem jeweils eine als Wirbelkammer 62a, 62b agierende Vertiefung, die durch eine ringförmige Vertiefung umgeben sind, welche einen Ringkanal 64a bilden. Von diesem Ringkanal 64a erstrecken sich jeweils zwei Einlasskanäle 66a in die Wirbelkammer 62a, 62b. Diese Einlasskanäle 66a verlaufen bezogen auf eine Wirbelhauptachse 4a bzw. 4b im Wesentlichen tangential, so dass das von der Ringkammer 64a in die jeweilige Wirbelkammer 62a, 62b einströmende Medium dort in eine rotative Bewegung umgelenkt wird. In Richtung des Rohres 30 sind die Wirbelkammern 62a, 62b und ein Teil des jeweiligen Ringkanals 64a durch eine Stirnfläche 30a des Rohres 30 verschlossen. Das durch den Zuführkanal 34 in Richtung der Düseneinheit 20 geförderte Medium kann am stiftförmigen Fortsatz 56 vorbei unmittelbar und durch den nicht verschlossenen Teil der Ringkanäle 64a zunächst nur in den jeweiligen Ringkanal 64a strömen. In Fig. 2c ist zur Erläuterung dessen mit gestrichelter Linie der Innendurchmesser des Rohres 30 an seiner Stirnfläche 30a dargestellt. Der Zugang zu den Ringkanälen ist innenseitig dieses Innendurchmessers gegeben. Von den Ringkanälen 64a gelangt das flüssige Medium durch die Einlasskanäle 66a in die Wirbelkammern 62a, 62b und wird dann durch die Austragöffnungen 60a, 60b in Form eines konischen Sprühstrahls abgegeben.

Die dargestellte Gestaltung erlaubt es, zwei Austragdüsen mit jeweils eigener Wirbelkammer 62a, 62b mit nur zwei Bauteilen 30, 50 zu realisieren. Zudem sind diese Bauteile 30, 50 aufgrund ihrer einfachen Formgebung sehr einfach herzustellen. Durch die Beabstandung der Wirbelkammern 62a, 62b zueinander entsprechend dem Durchmesser des Rohres 30 wird die auf Seiten des Aufsatzbauteils 50 einfach herstellbare Strömungsleitgeometrie durch die Stirnfläche 30a des Rohres 30 verschlossen, so dass der gewünschte Flüssigkeitspfad mit Wirbelkammer zwischen dem Zuführkanal 34 und den Austragöffnungen 60 hierdurch vervollständigt wird.

Wie insbesondere anhand von Fig. 2a zu erkennen ist, sind die Austragrichtungen 6a, 6b der beiden Austragöffnungen 60a, 60b divergierend zueinander ausgerichtet, um beispielsweise zwei separate Sprühstrahlen auszubilden, die beidseitig an einem Gaumen vorbei abgegeben werden. Aufgrund der zueinander parallelen Ausrichtungen der Wirbelkammerachsen 4a und 4b ist allerdings im Bereich der Auslassöffnungen eine Umlenkung erforderlich.

Die Fig. 2d verdeutlicht den Aufbau einer erfindungsgemäßen Düseneinheit nochmals in einer geschnittenen Darstellung.

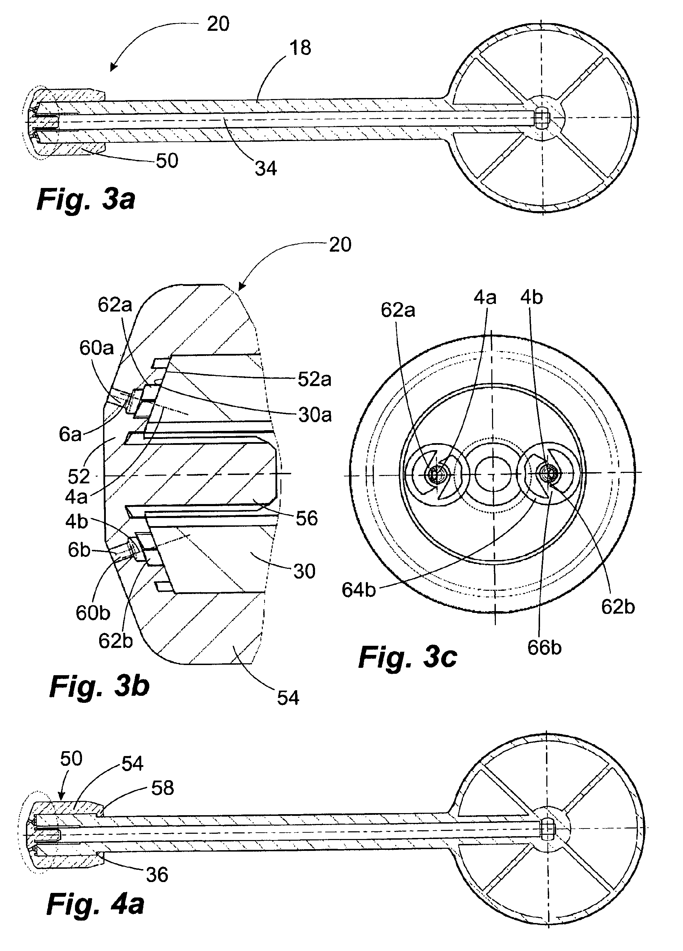

Bei der Ausgestaltung der Fig. 3a bis 3b wird das Maß dieser Umlenkung dadurch verringert, dass die Wirbelkammern 62a, 62b selbst angewinkelt ausgerichtet sind. Die Stirnfläche 30a des Rohres 30 ist hier im entsprechenden Winkel konusabschnittsförmig ausgebildet.

Bei der Ausgestaltung der Fig. 4a entspricht der Aufbau im Wesentlichen dem der Fig. 2a. Abweichend hiervon ist jedoch vorgesehen, dass das Aufsatzbauteil 50 nicht durch eine Pressverbindung oder durch Verschweißen am Rohr 30 befestigt ist, sondern durch eine formschlüssige Koppelung. Hierzu sind am Kragen 54 Rastvorsprünge 58 vorgesehen, die einen Absatz 36 am Rohr 30 hintergreifen.

## Patentansprüche

1. Düseneinheit (20) zum Austrag eines pharmazeutischen Mediums mit
- einem gemeinsamen Zuführkanal (34) zur Zuführung des Mediums und
- mindestens einer ersten und einer zweiten Austragdüse mit jeweils einer Austragöffnung (60) zum Austrag des durch den gemeinsamen Zuführkanal (34) zugeführten Mediums,
wobei
- die Düseneinheit (20) zur zerstäubten Ausbringung des Mediums ausgebildet ist und zum Zwecke der Zerstäubung eine der ersten Austragdüse (60a) zugeordnete erste Wirbelkammer (62a) und eine der zweiten Austragdüse (60b) zugeordnete zweite Wirbelkammer (62b) aufweist, wobei die Wirbelkammern (62a, 62b) jeweils als Kammern mit gegeneinander unbeweglichen Teilen ausgebildet sind, die mindestens einen Einlass und einen in Richtung der Austragöffnung (60) gerichteten Auslass aufweisen und deren Formgebung dergestalt ist, dass die Wirbelkammern (62a, 62b) das Medium mit einem Drall versehen, so dass das Medium in der jeweiligen Wirbelkammer um eine Wirbelkammerachse (4a, 4b) rotiert, die Düseneinheit (20) ein Basisbauteil aufweist, in welchem zumindest abschnittsweise der Zuführkanal (34) vorgesehen ist,
- die Düseneinheit mindestens ein Aufsatzbauteil (50) aufweist, welches die Austragöffnungen (60a, 60b) der ersten Austragdüse und der zweiten Austragdüse aufweist und
- die erste Wirbelkammer (62a) und die zweite Wirbelkammer (62b) durch Wandungen (30a) des Basisbauteils und durch Wandungen (52a) des Aufsatzbauteils (50) begrenzt wird, **dadurch gekennzeichnet, dass**
eine durch die erste Austragdüse definierte erste Austragrichtung (6a) und eine durch die zweite Austragdüse definierte zweite Austragrichtung (6b) einen Winkel >10° und <150° einschließen, wobei die Wirbelkammerachsen (4a, 4b) der Wirbelkammern (62a, 62b) der ersten und der zweiten Austragdüse einen hiervon abweichenden Winkel einschließen.

2. Düseneinheit (20) nach Anspruch 1
**dadurch gekennzeichnet, dass**
die Wirbelkammer (62b) zumindest der ersten Austragdüse eine Strömungsleitgeometrie aufweist, welche an einer Innenseite (52a) des Aufsatzbauteils (50) vorgesehen ist.

3. Düseneinheit (20) nach einem der vorstehenden Ansprüche,
**dadurch gekennzeichnet, dass**
das Basisbauteil zumindest in einem an das Aufsatzbauteil (50) angrenzenden Bereich durch einen Rohrabschnitt (30) gebildet ist, wobei eine ringförmige Stirnfläche (30a) dieses Rohrabschnitts (30) die erste und/oder die zweite Wirbelkammer (62a, 62b) begrenzt.

4. Düseneinheit (20) nach einem der vorsehenden Ansprüche,
**dadurch gekennzeichnet, dass**
die durch die erste Austragdüse definierte erste Austragrichtung und die durch die zweite Austragdüse definierte zweite Austragrichtung einen Winkel von mindestens 45° und maximal 90° einschließen.

5. Düseneinheit nach einem der vorstehenden Ansprüchen,
**dadurch gekennzeichnet, dass**
zumindest der erste Wirbelkammer zumindest der ersten Austragdüse ein Einlasskanal zugeordnet sind, der in die erste Wirbelkammer mündet.

6. Düseneinheit (20) nach einem der vorstehenden Ansprüchen,
**dadurch gekennzeichnet, dass**
zumindest der erste Wirbelkammer (62a) zumindest der ersten Austragdüse zumindest zwei Einlasskanäle (66a) zugeordnet sind, die in die erste Wirbelkammer (62a) münden und die vorzugsweise von einem die Wirbelkammer (62a) umgebenden Ringkanal (64a) abgehen, der vom Zuführkanal (34) gespeist wird.

7. Spender (10) zum Austrag von pharmazeutischer Medien in zerstäubter Form,
**dadurch gekennzeichnet, dass**
der Spender eine Düseneinheit (20) nach einem der vorstehenden Ansprüche aufweist.

8. Spender (10) nach Anspruch 7,
**dadurch gekennzeichnet, dass**
der Spender als Rachensprühspender ausgebildet ist und zu diesem Zweck einen rohrartigen Abschnitt (30) von mindestens 20 mm Länge zur Einführung in den Mund eines Patienten aufweist, wobei der rohrartige Abschnitt (30) vorzugsweise das Basisbauteil der Düseneinheit (20) bildet.

9. Spender (10) nach Anspruch 7 oder 8,
**dadurch gekennzeichnet, dass**
der Spender (10) mit einem Medium zur Behandlung von Erkältungskrankheiten befüllt ist.

## Claims

1. A nozzle unit (20) for discharge of a pharmaceutical medium including
- a common supply channel (34) for supplying the medium, and
- at least a first and a second discharge nozzle each including a discharge orifice (60) for discharge of the medium supplied through the common supply channel (34),
wherein
- the nozzle unit (20) is configured for atomized delivery of the medium and includes a first vortex chamber (62a) assigned to the first discharge nozzle (60a) and a second vortex chamber (62b) assigned to the second discharge nozzle (60b) for the purpose of atomizing, wherein the vortex chambers (62a, 62b) each are chambers defined by parts that are immobile one relative to the other, including at least one inlet and one outlet aligned in the direction of the discharge orifice (60) and having a shape design such that the vortex chambers (62a, 62b) induce a twist in the medium so that the medium in the respective vortex chamber rotates about a vortex chamber axis (4a, 4b), the nozzle unit (20) includes a basic component in which the supply channel (34) is provided at least in sections,
- the nozzle unit includes at least one top component (50) which includes the discharge orifices (60a, 60b) of the first discharge nozzle and the second discharge nozzle, and
- the first vortex chamber (62a) and the second vortex chamber (62b) are delimited by walls (30a) of the basic component and by walls (52a) of the top component (50),
**characterized in that**
a first discharge direction (6a) defined by the first discharge nozzle and a second discharge direction (6b) defined by the second discharge nozzle form an angle of more than 10° and less than 150°, wherein the vortex chamber axes (4a, 4b) of the vortex chambers (62a, 62b) of the first and the second discharge nozzles enclose an angle different therefrom.

2. The nozzle unit (20) according to claim 1, **characterized in that** the vortex chamber (62a) of at least the first discharge nozzle includes a flow deflecting geometry which is provided on an interior side (52a) of the top component (50).

3. The nozzle unit (20) according to any one of the preceding claims, **characterized in that** the basic component at least in a zone adjacent to the top component (50) is formed by a tubular section (30), wherein an annular end face (30a) of said tubular section (30) delimits the first and/or the second vortex chambers (62a, 62b).

4. The nozzle unit (20) according to any one of the preceding claims, **characterized in that** a first discharge direction defined by the first discharge nozzle and a second discharge direction defined by the second discharge nozzle form an angle of at least 45° and maximum 90°.

5. The nozzle unit according to any one of the preceding claims, **characterized in that** at least the first vortex chamber of at least the first discharge nozzle is assigned an inlet channel which ends in the first vortex chamber.

6. The nozzle unit (20) according to any one of the preceding claims, **characterized in that** at least the first vortex chamber (62a) of at least the first discharge nozzle is assigned at least two inlet channels (66a) which end in the first vortex chamber (62a) and preferably are branched off from an annular channel (64a) surrounding the vortex chamber (62a), said annular channel being fed by the supply channel (34).

7. A dispenser (10) for discharge of pharmaceutical media in an atomized condition, **characterized in that** the dispenser includes a nozzle unit (20) according to any one of the preceding claims.

8. The dispenser (10) according to claim 7, **characterized in that** the dispenser is configured to be a pharyngeal spray dispenser and for that purpose includes a tubular section (30) of at least 20 mm in length, to be introduced into the mouth of a patient, wherein said tubular section (30) is preferably the basic component of the nozzle unit (20).

9. The dispenser (10) according to claim 7 or 8, **characterized in that** the dispenser (10) is filled with a medium adapted to treatment of common cold diseases.

## Revendications

1. Unité à buses (20) pour la distribution d'un produit pharmaceutique, comprenant
- un canal d'alimentation commun (34) pour l'acheminement du produit et
- au moins une première et une deuxième buse de distribution présentant respectivement une ouverture de distribution (60) pour la distribution du produit acheminé à travers le canal d'alimentation commun (34),
- l'unité à buses (20) étant réalisée pour la distribution sous forme pulvérisée du produit et comprenant, en vue de la pulvérisation, une première chambre à turbulence (62a) associée à la première buse de distribution (60a) et une deuxième chambre à turbulence (62b) associée à la deuxième buse de distribution (60b), les chambres à turbulence (62a, 62b) étant réalisées respectivement en tant que chambres présentant des parties non mobiles les unes par rapport aux autres, lesquelles comprennent au moins une entrée et une sortie orientée en direction de l'ouverture de distribution (60) et dont la configuration est telle que les chambres à turbulence (62a, 62b) dotent le produit d'une turbulence, de telle sorte que le produit dans la chambre à turbulence respective tourne autour d'un axe de chambre à turbulence (4a, 4b), l'unité à buses (20) comprenant un composant de base dans lequel le canal d'alimentation (34) est prévu au moins en partie,
- l'unité à buses comprenant au moins un composant rapporté (50), lequel comprend les ouvertures de distribution (60a, 60b) de la première buse de distribution et de la deuxième buse de distribution et
- la première chambre à turbulence (62a) et la deuxième chambre à turbulence (62b) étant délimitées par des parois (30a) du composant de base et par des parois (52a) du composant rapporté (50), **caractérisée en ce**
**qu'**une première direction de distribution (6a) définie par la première buse de distribution et une deuxième direction de distribution (6b) définie par la deuxième buse de distribution forment un angle >10° et <150°, les axes de chambre à turbulence (4a, 4b) des chambres à turbulence (62a, 62b) de la première et de la deuxième buse de distribution formant un angle différent de celui-ci.

2. Unité à buses (20) selon la revendication 1,
**caractérisée en ce que**
la chambre à turbulence (62b) d'au moins la première buse de distribution présente une géométrie de guidage d'écoulement, laquelle est prévue sur un côté intérieur (52a) du composant rapporté (50).

3. Unité à buses (20) selon l'une quelconque des revendications précédentes,
**caractérisée en ce que**
le composant de base est formé par une portion tubulaire (30) au moins dans une région adjacente au composant rapporté (50), une surface frontale annulaire (30a) de cette portion tubulaire (30) délimitant la première et/ou la deuxième chambre à turbulence (62a, 62b).

4. Unité à buses (20) selon l'une quelconque des revendications précédentes,
**caractérisée en ce que**
la première direction de distribution définie par la première buse de distribution et la deuxième direction de distribution définie par la deuxième buse de distribution forment un angle d'au moins 45° et d'au maximum 90°.

5. Unité à buses selon l'une quelconque des revendications précédentes,
**caractérisée en ce**
**qu'**un canal d'entrée est associé à au moins la première chambre à turbulence d'au moins la première buse de distribution, lequel canal d'entrée débouche dans la première chambre à turbulence.

6. Unité à buses (20) selon l'une quelconque des revendications précédentes,
**caractérisée en ce**
**qu'**au moins deux canaux d'entrée (66a) sont associés à au moins la première chambre à turbulence (62a) d'au moins la première buse de distribution, lesquels canaux d'entrée débouchent dans la première chambre à turbulence (62a) et partent de préférence d'un canal annulaire (64a) entourant la chambre de turbulence (62a), lequel est alimenté par le canal d'alimentation (34).

7. Distributeur (10) pour la distribution de produits pharmaceutiques sous forme pulvérisée,
**caractérisé en ce que**
le distributeur comprend une unité à buses (20) selon l'une quelconque des revendications précédentes.

8. Distributeur (10) selon la revendication 7,
**caractérisé en ce que**
le distributeur est réalisé sous forme de distributeur pulvérisateur pour la gorge et comprend à cet effet une portion (30) de type tube d'au moins 20 mm de long destinée à être introduite dans la bouche d'un patient, la portion (30) de type tube formant de préférence le composant de base de l'unité à buses (20).

9. Distributeur (10) selon la revendication 7 ou 8,
**caractérisé en ce que**
le distributeur (10) est rempli d'un produit pour le traitement de maladies dues à un refroidissement.
